# EUROPÄISCHE PATENTSCHRIFT

(11) **EP 2 424 835 B1**
(45) Veröffentlichungstag und Bekanntmachung des Hinweises auf die Patenterteilung: **01.07.2015**
(21) Anmeldenummer: 10715721.6
(22) Anmeldetag: 27.04.2010
(51) Int. Cl.: C07C 319/20, C07C 323/58, C07D 209/30, C07D 209/34

(54) **VERFAHREN ZUR HERSTELLUNG VON OXINDOLEN UND ORTHO-SUBSTITUIERTEN ANILINEN**
METHOD FOR PRODUCING OXINDOLES AND ORTHO-SUBSTITUTED ANILINES
PROCÉDÉ DE FABRICATION D'OXINDOLES ET D'ANILINES ORTHO-SUBSTITUÉS

(30) Priorität: 02.05.2009 EP 09006050
(43) Veröffentlichungstag der Anmeldung: 07.03.2012
(73) Patentinhaber: Bayer Intellectual Property GmbH, 40789 Monheim am Rhein (DE)
(72) Erfinder: FORD, Mark, James, 61389 Schmitten (DE); KARIG, Gunter, 65719 Hofheim am Taunus (DE)
(74) Vertreter: BIP Patents
(86) Internationale Anmeldenummer: PCT/EP2010/002566
(87) Internationale Veröffentlichungsnummer: WO 2010/127786

(56) Entgegenhaltungen:
- EP-A1- 1 213 290
- US-A- 3 972 894
- WRIGHT S W; MCCLURE L D; HAGEMAN D L: "A Convenient Modification of the Gassman Oxindole Synthesis" TETRAHEDRON LETTERS, ELSEVIER, AMSTERDAM, Bd. 37, Nr. 27, 1. Juli 1996 (1996-07-01), Seiten 4631-4634, XP004028978 ISSN: 0040-4039 in der Anmeldung erwähnt
- P.G. GASSMAN ET AL: "Oxindoles. A new, general method of synthesis" J.AM.CHEM.SOC., Bd. 96, Nr. 17, 21. August 1974 (1974-08-21), Seiten 5508-5512, XP002545536 in der Anmeldung erwähnt
- I. DELIMOGE ET AL: "Simple synthetic routes to 5-(3,6-dihydro-2-oxo-2H-1,3,4-thiadiazin-5 -yl)-1H-indole-2,3-diones and their derivatives" J. HETEROCYCLIC CHEM., Bd. 28, 1991, Seiten 1525-1532, XP002545537
- D.A. WALSH: "Antiinflammatory agents. 3. Synthesis and pharmacological evaluation of 1-amino-3-benzoylphenylacetic acid and analogues" J. MED. CHEM., Bd. 27, Nr. 11, 1. November 1984 (1984-11-01), Seiten 1379-1388, XP002545538
- CONNOLLY T J; DURST T: "Metal hydride mediated reduction of 1,3-dimethyl-3(methylthio)oxindole" CANADIAN JOURNAL OF CHEMISTRY, NATIONAL RESEARCH COUNCIL. OTTAWA, CA, Bd. 75, 1. Januar 1997 (1997-01-01), Seiten 542-546, XP002384186 ISSN: 0008-4042
- T.J. CONNOLLY ET AL: "Non-reductive desulfenylation of 3-thioalkyl-2-oxindoles" SYNLETT, 1. Juli 1996 (1996-07-01), Seiten 663-664, XP002545539

## Beschreibung

Die Erfindung betrifft das technische Gebiet der chemischen Synthese von biologisch aktiven Verbindungen, vorzugsweise für Zwischenprodukte für die Synthese von Feinchemikalien und Wirkstoffen aus der Pharmazie und/oder der Landwirtschaft.

Prinzipiell gehört der selektive Austausch von Wasserstoff an einem aromatischen System mit einem substituierten Kohlenstoff-Atom zu einer der fundamentalen Umsetzungen in der organischen Chemie und ist damit bekannt.

Eine Klasse von Verbindungen, die so hergestellt werden können, sind z.B. optional substituierte 3-Alkylthioindol-2-one (3-(Alkylsulfanyl)-1,3-dihydro-2H-indol-2-one), die wiederum in optional substituierte 2-Oxindole (1,3-Dihydro-2H-indol-2-one) umgesetzt werden können. Optional substituierte Oxindole und deren Vorprodukte, wie optional substituierte 3-Alkylthioindol-2-one, sind vielseitig verwendbare Zwischenprodukte für Wirkstoffsynthesen (Bioorg. Med. Chem. Lett. 2006, 16, 2109; JP 2008-101014; WO 96/41799 A1). Weitere Verwendung als Vorstufen zu pharmazeutischen Verbindungen sind beschrieben in: US 2005/0090541 A1, EP 636608 A, US 4690943 A.

Die meisten der beschriebenen, unterschiedlichen Synthesen von Oxindolen verwenden eine Variation einer Friedel-Crafts-Reaktion (Stolle Synthesis, W.C. Sumpter, Chem. Rev. 1945, 37, 443-449). Jedoch sind Stolle-Synthesen nur bedingt einsetzbar, da sie stark saure Bedingungen und ein elektronenreiches Anilin benötigen. Daneben sind aber auch Radikal-, Nitrenium-ion- und Organolithium-Reaktionen sowie photochemisch abhängige Methoden bekannt. Diese sind aber auch limitiert durch den herzustellenden Typ von Oxindolen, die Kompatibilität von Substraten, den Reaktionsbedingungen sowie durch den Umstand, dass der Aromat schon einen Halogen-Substituenten besitzen muss, welcher dann ersetzt wird. (Radikal-Verfahren: Zard et al., Tetrahedron Lett. 1994, 35, 9553-9556; Zard et al., Tetrahedron Lett. 1994, 35, 1719-1722; Jones et al., Tetrahedron Lett. 1994, 35, 7673-7676; Kikugawa et al., Chem. Letters 1987, 1771-1774; Clark et al., Synthesis 1991, 871-878; Yonemitsu et. al., Chem. Pharm. Bull. 1981, 29, 128-136; s. Schema 1).

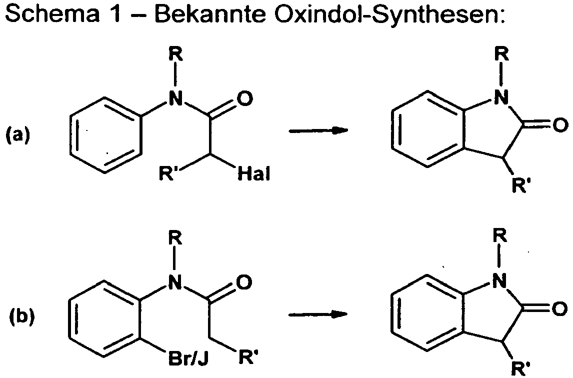

Das Verfahren von Gassman et al. (Organic Synthesis Coll., Vol. 6, 601 und Vol. 56, 72), welches von Anilin und Methylthioacetat-Ester über Chlorierung und Behandlung mit Triethylamin bei -70°C abläuft, erscheint geeignet in Sinne von Durchführbarkeit, Verfügbarkeit von Edukten, kurzer Reaktionsgeschwindigkeit und Reproduzierbarkeit. Jedoch wird aber auch beschrieben, dass gute Ausbeuten nur erzielt werden können, wenn die instabilen N-Chlor- (1) oder N-Sulfonium- (2) Zwischenprodukte unter -65°C, im Normalfall bei -78°C gebildet werden (Gassman et. al., J. Am. Chem. Soc., 1974, 96(17), 5508; Gassman et al., J. Am. Chem. Soc., 1974, 96(17), 5512; WO 96/41799 A1; s. Schema 2).

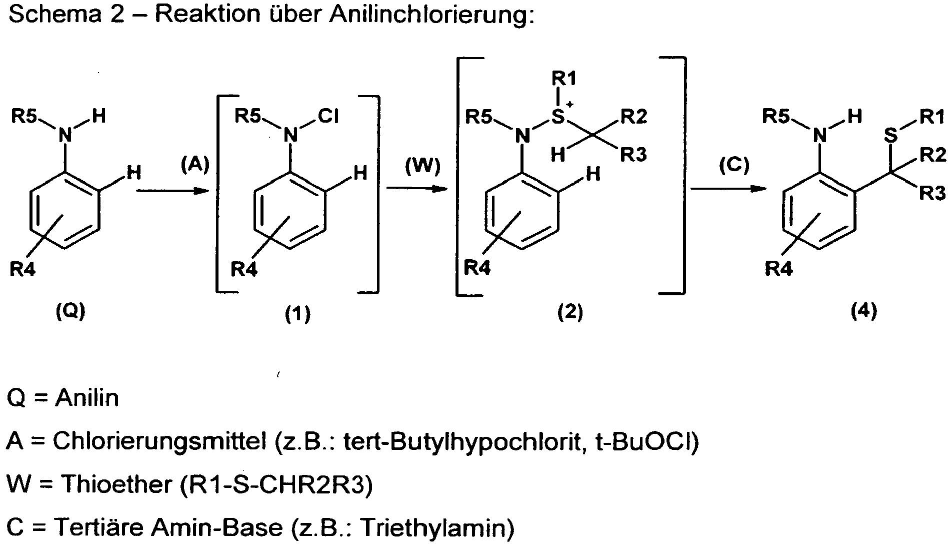

Das Chlorierungsmittel der Wahl gemäß Literatur ist das instabile und explosive tert-Butylhypochlorit, da das Nebenprodukt der Chlorierung dann den neutralen tert-Butylalkohol ergibt. In den wenigen Fällen, worin Sulfurylchlorid (SO₂Cl₂) eingesetzt worden war, wurde eine zweite, nicht nukleophile Base, wie "proton sponge", eingesetzt (Johnson, J. Org. Chem. 1990, 55, 1374; Warpehoski, Tetrahedron Lett. 1986, 27, 4103). Da beide Varianten bei tiefen Temperaturen durchgeführt werden, ist dies jedoch keine gangbare Lösung im technischen (industriellen) Maßstab.

Wright et al. (Tetrahedron Lett. 1996, 37, 4631) beschreiben eine Alternative, wobei das Chlorsulfonium-Zwischenprodukt (3) aus einem Sulfoxid und Oxalylchlorid hergestellt wurde (s. Schema 3). Hierbei ist das Chlorsulfonium-Zwischenprodukt (3) ebenfalls instabil. Für diese Reaktion muss das Sulfoxid zuerst hergestellt und isoliert werden. Aus Gründen der Stabilität muss die Reaktion bei -78°C ablaufen und um eine Reaktion zwischen Anilin und Oxalylchlorid zu vermeiden, wird die Reaktion stufenweise durchgeführt.

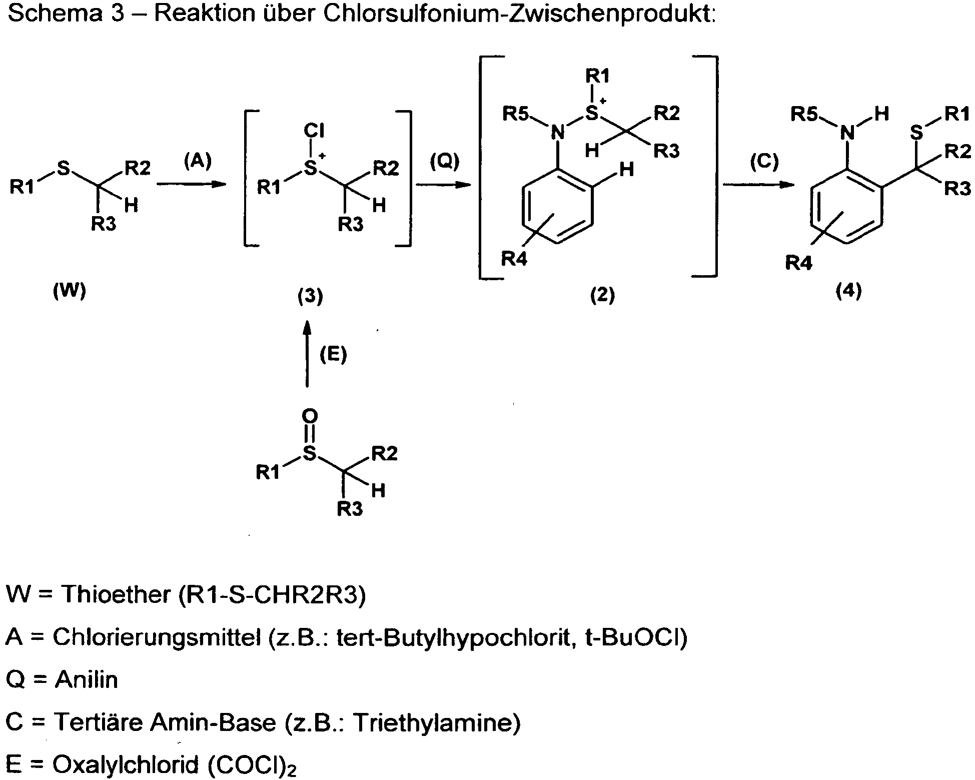

Die nach diesen Verfahren (Schema 2 oder Schema 3) beschriebenen Verbindungen (4) lassen sich mechanistisch bedingt nur über die Verbindungen (2) herstellen. Zwangsläufig ist dabei die Verwendung einer Base (C) zur Umlagerung zu den Verbindungen (4) notwendig. In der Literatur wird dazu eine zusätzliche Base zum Anilin (Verbindungen der Formel Q) wie "proton sponge" oder Triethylamin genannt. Die in diesen Verfahren verwendete zusätzliche Base muss zwangsläufig bei Synthesen im technischen (industriellen) Maßstab zurückgewonnen und von den nicht abreagierten Verbindungen der Formel Q getrennt werden, um die reisolierten Verbindungen der Formel Q wieder als Startmaterialien verwenden zu können.

Die Gründe, warum die Reaktion so empfindlich gegenüber Reaktionstemperaturen über -70°C ist und warum sie immer stufenweise durchgeführt wurde, sind vielfältig. Zuerst einmal kommen die funktionellen Gruppen, die an der Reaktion teilnehmen, d.h. das Stickstoff-Atom des Anilins und das Schwefel-Atom des Thioethers, unverändert sowohl im Produkt (4) als auch im Edukt vor. Somit wäre eine selektive Chlorierung während der Reaktion nicht zu erwarten, in der das Produkt (4) gleich gebildet wird. Aus diesem Grunde verwenden alle literaturbekannten Methoden eine stufenweise Reaktion.

Daneben kann sich bei höheren Temperaturen als -65°C das N-Chloranilin in einen im Kern chlorierten Aromaten umwandeln, wie auch andere Oxidationsprodukte (Dimere) bilden. Somit ist es nicht überraschend, dass das deutlich weniger elektronenreiche und somit deutlich weniger zur Kern-Chlorierungreaktive Acetanilid nur Kern-Chlorierungen mit tert-Butylhypochlorit bei 0°C bildet (Lengyel et. al. Synth. Comm., 1998, 28 (10), 1891-1896).

Weiterhin können die Sulfonium-Zwischenprodukte (2) oder (3) in Gegenwart von Basen eliminieren und das reaktive Nebenprodukt (5) bilden, das z.B. mit einem Anilin kondensieren würde, wodurch die Nebenkomponente (6) irreversibel erzeugt wird (s. Schema 4). Dies entspricht der sog. Pummerer-Oxidation des R2-CH-R3-Restes.

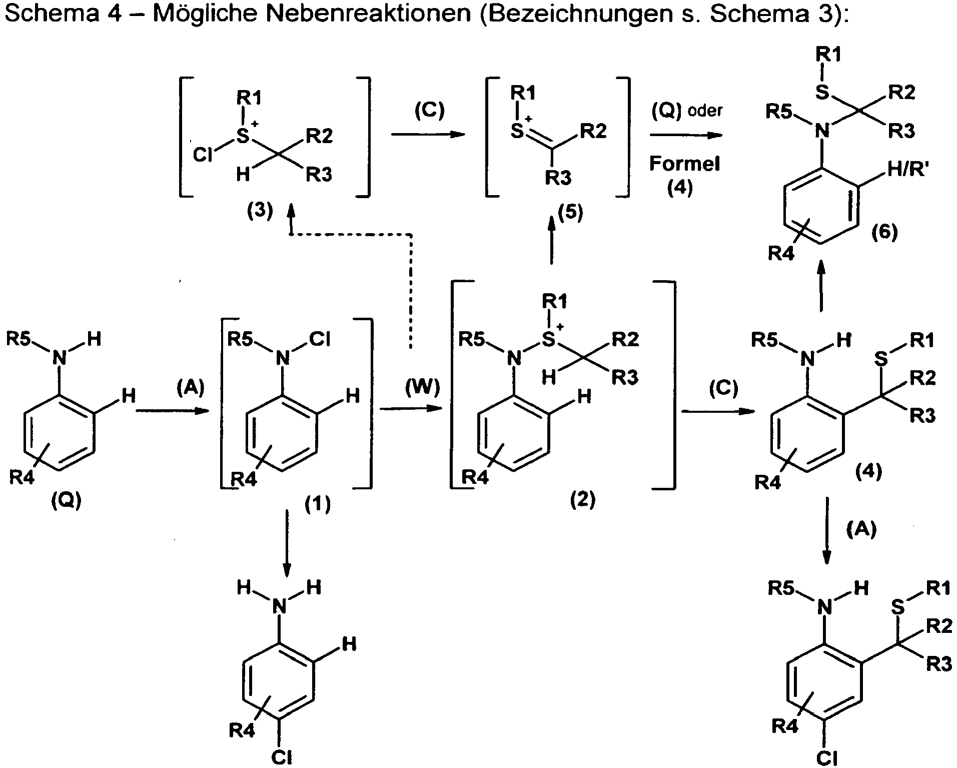

EP1213290 offenbart die Herstellung von 6,7-Dimethyl-5-methoxy-3-(methylthio)-1,3-dihydro-2H-indole-2-one ausgehend von 4-methoxy-2,3-dimethylaniline. Dabei wird zu einer Lösung von Methyl (methylthio)acetat in Dichloromethane Sulfurylchlorid bei einer Temperatur von -78 °C zugegeben. Danach wird eine Lösung von 4-Methoxy-2,3-dimethylaniline und ein Protonenschwamm tropfenweise zugegeben. In einem nächsten Schritt wird dann Triethylamin zugegeben.

Es bestand daher die Aufgabe, ein modifiziertes Verfahren bereitzustellen, das eine im Vergleich zu den oben genannten Verfahren verbesserte Herstellung von Verbindungen (4) über Zwischenprodukte (2) (Sulfoniumsalze) im technischen Maßstab ermöglicht mit Vorteilen wie verbesserter Gesamtausbeute und/oder Produktreinheit, verringertem Einsatz von Ausgangsmaterialien, Verzicht auf weitere Hilfstoffe (wie z.B. eine zweite Base) oder vereinfachtem Verfahrensablauf(wie z.B. Reaktionen bei höherer Temperatur) oder Verwendung technisch (industriell) geeigneterer Lösungsmittel (weniger giftig, besser wiedergewinnbar).

Die so hergestellten Verbindungen (4) sollten vorzugsweise auch eine Weiterverarbeitung zu Oxindolen (1,3-Dihydro-2H-indol-2-one) erlauben, die ebenfalls Zwischenprodukte für die Synthese von Feinchemikalien und Wirkstoffen aus der Pharmazie und/oder der Landwirtschaft sein können.

Überraschend wurde nun gefunden, dass trotz der oben genannten Probleme die Reaktion so modifiziert werden konnte, dass sie in einem industriell realisierbaren Maßstab durchgeführt werden kann.

Gegenstand der Erfindung ist ein Verfahren zur Herstellung von Verbindungen der Formel (4): 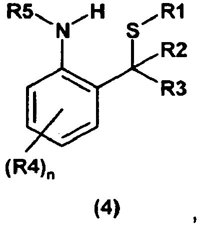 worin
R1 = C1-C6 Alkyl, substituiertes Alkyl, Aryl oder substituiertes Aryl, vorzugsweise C1-C4 Alkyl;
R2 = H, C1-C6 Alkyl oder substituiertes Alkyl;
R3 = elektronenziehende oder aktivierende Gruppe, ausgewählt aus -CO-R1; -CO-X,
   wobei X = OR1, SR1, NR2R2', wobei R2' wie R2 definiert ist und gleich oder anders als R2 sein kann; R2 und R2' können einen Ring bilden; SO(n')-R1, wobei n' = 0, 1, oder 2 sein kann; -CN; -NO₂, Aryl or Heteroaryl;
R4 = F, Cl, Br, I, CF₃, CN, NO₂, COX;
   wobei X = OR1, SR1, NR2R2', wobei R2' wie R2 oben definiert ist und gleich oder anders als R2 sein kann, vorzugsweise F oder Cl, insbesondere 2-F;
n = 1 - 4, vorzugsweise 1 - 2 , insbesondere 1;
R5 = H, C1-C6 Alkyl oder substituiertes Alkyl,
bedeutet,
dadurch gekennzeichnet, dass man eine Mischung
eines Anilins (Verbindung der Formel Q): 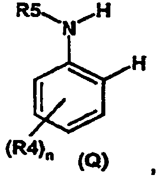 worin die Reste R4, n und R5 wie in Formel (4) definiert sind,
mit einem Thioether (Verbindung der Formel W): 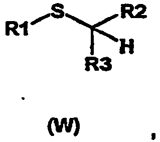 worin die Reste R1, R2 und R3 wie in Formel (4) definiert sind,
in Gegenwart von Sulfurylchlorid (SO₂Cl₂) als Chlorierungsmittels und eines organischen Lösungsmittels bei einer Reaktionstemperatur im Bereich zwischen -60 und -10°C, insbesondere zwischen -50 und -20°C, zur Verbindung der Formel (4) über das mechanistisch bedingte Zwischenprodukt der Formel (2) umsetzt.

Die hieraus resultierende selektive und saubere Reaktion mit guten Ausbeuten ist besonders überraschend und steht im Gegensatz zur Lehrmeinung, wonach beschrieben wird, dass gute Ausbeuten nur erzielt werden können, wenn die instabilen N-Chlor- (1) oder N-Sulfonium- (2) Zwischenprodukte (s. Schema 2) unter -65°C, im Normalfall bei -78°C gebildet werden (Gassman et. al., J. Am. Chem. Soc., 1974, 96(17), 5508; Gassman et al., J. Am. Chem. Soc., 1974, 96(17), 5512; WO 96/41799 A1).

Der im erfindungsgemäßen Verfahren verwendete Thioether (Verbindung der Formel W) hat die folgende Struktur: 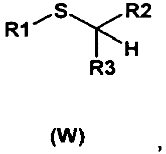 wobei
R1 = C1-C6 Alkyl, substituiertes Alkyl, Aryl oder substituiertes Aryl, vorzugsweise C1-C4 Alkyl;
R2 = H, C1-C6 Alkyl oder substituiertes Alkyl;
R3 = elektronenziehende oder aktivierende Gruppe, ausgewählt aus -CO-R1; -CO-X,
   wobei X = OR1, SR1, NR2R2', wobei R2' wie R2 definiert ist und gleich oder anders als R2 sein kann; R2 und R2' können einen Ring bilden;
   SO(n')-R1, wobei n' = 0, 1, oder 2 sein kann; -CN; -NO₂, Aryl or Heteroaryl;
bedeutet.

Soweit im Folgenden auf Thioether verwiesen wird, ist die oben genannte Verbindung gemeint.

Das im erfindungsgemäßen Verfahren verwendete Anilin (Verbindung der Formel Q) hat die folgende Struktur: 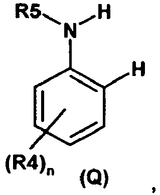 wobei
R4 = F, Cl, Br, I, CF₃, CN, NO₂, COX;
   wobei X = OR1, SR1, NR2R2', wobei R2' wie R2 oben definiert ist und gleich oder anders als R2 sein kann, vorzugsweise F oder Cl, insbesondere 2-F;
n = 1 - 4, vorzugsweise 1 - 2 , insbesondere 1;
R5 = H, C1-C6 Alkyl oder substituiertes Alkyl,
bedeutet.

Zwingend ist, dass ein Wasserstoffatom in ortho-Position zum Stickstoffatom des Anilins vorliegt. Der Ring kann mit einem oder mehreren Substituenten R4 substituiert sein. Soweit im Folgenden auf Anilin verwiesen wird, ist die oben genannte Verbindung gemeint.

Als Chlorierungsmittel wird Sulfurylchlorid (SO₂Cl₂) verwendet, welches überraschend gegenüber der Literaturmeinung HCl generiert, ohne dass eine zweite, nicht nucleophile Base eingesetzt werden muss.

Die Reaktion im erfindungsgemäßen Verfahren kann mit verschiedenen Lösungsmitteln durchgeführt werden.

So können unpolare organische Lösungsmittel eingesetzt werden, wie Chloralkane (beispielsweise Dichlormethan und Dichlorethan), Aromaten (beispielsweise Benzol, Toluol, Xylol), Haloaromaten (beispielsweise Chlorbenzol, Dichlorbenzol), substituierte Aromaten (beispielsweise Benzotriflurid, Chlorbenzotrifluorid, Chlortoluol, Chlorxylol) allein oder als Mischungen untereinander oder als Mischungen mit Alkanen und Cycloalkanen.

Daneben sind aber auch polare organische Lösungsmittel geeignet. Dies ist entgegen der Lehre von Gassman (J. Am. Chem. Soc., 1972, 94, 3891), wonach N-Chlor-Derivate in polaren Lösungsmittel weniger stabil sind. So kann die Reaktion im erfindungsgemäßen Verfahren vorzugsweise in einem Ester-Lösungsmittel, wie zum Beispiel C1-C6 Alkylacetat (beispielsweise Methylacetat, Ethylacetat, n-Propylacetat, iso-Propylacetat, 2-Methyl-Prop-1-ylacetat, n-Butylacetat, But-2-ylacetat, Pentylacetate, Hexylacetate und Cyclalkylacetate, C1-C6 Alkyl und Cycloalkylpropionate, C1-C6 Alkyl und Cycloalkyl -n-butyrate, -iso-butyrate, - pentanoate und -hexanoate und -cyclopentanoate und -cyclohexanoate) oder in Mischungen davon oder in Mischung mit anderen Lösungsmitteln, durchgeführt werden. Ester-Lösungsmittel haben gegenüber anderen Lösungsmitteln, wie z.B. dem in der Literatur verwendete Dichlormethan, den Vorteil, dass sie technisch (industriell) geeigneter sind (weniger giftig, besser wiedergewinnbar).

Das erfindungsgemäße Verfahren zur Herstellung von Verbindungen der Formel (4), basiert darauf, dass die beiden Verbindungen Anilin (Verbindung der Formel Q) und Thioether (Verbindung der Formel W) vorteilhaft vorgemischt werden können.

Die hieraus resultierende selektive und saubere Reaktion ist besonders überraschend und steht im Gegensatz zur Lehrmeinung, wonach Gassman demonstrierte, dass je nach Substituent eine N-Chlorierung oder eine S-Chlorierung optimal für die Reaktion ist (Gassman, J. Am. Chem. Soc., 1974, 96(17), 5512). Weiterhin würde man von einer Mischung, bestehend aus Anilin und Thioether, keine Selektivität bei einer Chlorierung erwarten, warum nach bisheriger Lehrmeinung nur stufenweise gearbeitet wurde.

Für gute Produktausbeuten hat sich gezeigt, dass es vorteilhaft ist, bis zu 1 Äquivalent, bevorzugt 0,5 bis 1,0 Äquivalente, besonders bevorzugt 0,7 bis 1,0 Äquivalente, insbesondere bevorzugt 0,8 bis 0,95 Äquivalente des Chlorierungsmittels zu verwenden. Das Chlorierungsmittel wird zu einer Mischung eines Äquivalents des Thioethers (Verbindung der Formel W) und eines technisch ökonomischen Überschusses des Anilins (Verbindung der Formel Q) von 2,0 bis 5,0 Äquivalente, bevorzugt 2,0 bis 3,0 Äquivalente, besonders bevorzugt 2,0 bis 2,5 Äquivalente zugegeben.

Das Chlorierungsmittel kann vorzugsweise mit einem Lösungsmittel oder einem Lösungsmittelgemisch vorverdünnt werden und kann vorzugsweise vorgekühlt werden.

Eine besondere Ausführungsform der Erfindung ist, dass für gute Produktausbeuten auch eine Teilmenge des Anilins (zwischen 1 und 99 Gew.%, bevorzugt zwischen 20 und 80 Gew.%, besonders bevorzugt 30 bis 70 Gew.% der Gesamtmenge an Anilin) getrennt vom, aber simultan oder teilweise simultan mit dem Chlorierungsmittel, zugegeben werden.

Eine besondere Ausführungsform der Erfindung im Hinblick auf einen vereinfachten (industriellen) Verfahrensablauf ist, dass bei dem erfindungsgemäßen Verfahren zur Herstellung von Verbindungen der Formel (4) auf die Zugabe eines zusätzlichen tertiären Amins verzichtet werden kann, wodurch die Wiederverwendung des nicht abreagierten, reisolierten Anilins (Verbindung der Formel Q) vereinfacht wird.

So wurde gefunden, dass ein Überschuss an den überraschenderweise elektronenarmen Anilinen als milde Base bei der Bildung von Produkt der Formel (4) fungiert. Dies ist überraschend gegenüber der Standard-Gassman-Reaktion, welche die Zugabe eines zusätzlichen tertiären Amins verwendet (vgl. Schemata 2 und 3: C = Tertiäre Amin-Base, z.B.: Triethylamin). Das Anilin ist offensichtlich in der Lage, die Umlagerung zum Produkt der Formel (4) zu katalysieren und daher auch HCl von einem Chlorsulfonium-Zwischenprodukt der Formel (3) zu eliminieren, welches nur zu Nebenreaktionen führen würde. Trotzdem und gerade deswegen ist es sehr überraschend, dass die Reaktion so durchgeführt werden kann. Gleichzeitig ist der Verzicht auf ein zusätzliches tertiäres Amin in dem erfindungsgemäßen Verfahren vorteilhaft, da auf eine spätere aufwendige Trennung von zurückgewonnenem Anilin und tertiärem Amin verzichtet werden kann.

Gegenstand der Erfindung ist auch das Verfahren zur Herstellung von Verbindungen der Formeln (7) und (8): 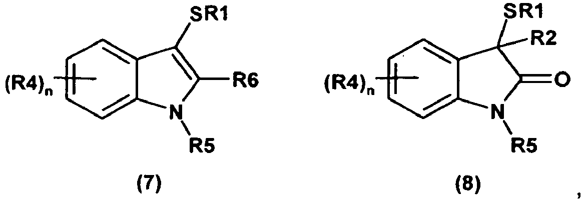 worin
R1 = C1-C6 Alkyl, substituiertes Alkyl, Aryl oder substituiertes Aryl,
   vorzugsweise C1-C4 Alkyl;
R2 = H, C1-C6 Alkyl oder substituiertes Alkyl;
R4 = F, Cl, Br, I, CF₃, CN, NO₂, COX;
   wobei X = OR1, SR1, NR2R2', wobei R2' wie R2 oben definiert ist und gleich oder anders als R2 sein kann, vorzugsweise F oder Cl, insbesondere 2-F;
n = 1 - 4, vorzugsweise 1 - 2 , insbesondere 1;
R5 = H, C1-C6 Alkyl oder substituiertes Alkyl;
R6 = C1-C6 Alkyl oder substituiertes Alkyl, vorzugsweise C1-C4 Alkyl oder OH;
bedeutet,
wobei Verbindungen der Formel (4): 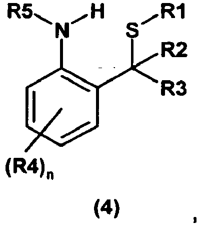 mit den oben genannten Definitionen der Reste R1, R2, R4 und R5 und wobei R3 eine elektronenziehende oder aktivierende Gruppe, wie -CO-R1;-CO-X, wobei X = OR1, SR1, NR2R2', wobei R2' wie R2 definiert ist und gleich oder anders als R2 sein kann; R2 und R2' können einen Ring bilden;
SO(n')-R1, wobei n' = 0, 1, oder 2 sein kann; -CN; -NO₂, Aryl or Heteroaryl; bedeutet,
hergestellt nach dem oben beschriebenen erfindungsgemäßen Verfahren zur Herstellung von Verbindungen der Formel (4),
ohne Isolierung der Verbindungen der Formel (4), gegebenenfalls In Gegenwart eines Säure-Katalysators, zu dem Indol der Formel (7) oder dem Oxindol der Formel (8) umgesetzt werden.

Im Falle von R3 = CO-R1 erhält man das Indol der Formel (7), während man im Falle von R3 = COX das Oxindol der Formel (8) oder das Indol der Formel (7) mit R6 = OH erhält. Daher sind im Sinne der vorliegenden Erfindung diese spezifischen Indole auch mit dem Begriff "Oxindol" umfasst.

Für den Säure-Katalysator im erfindungsgemäßen Verfahren können mineralische oder organische Säuren wie H⁺X⁻, wobei X⁻ = F⁻, Cl⁻, Br, I⁻, HSO₄⁻, BF₄⁻, H₂PO₄⁻, SO₄²⁻, HPO₄²⁻, PO₄³⁻, R'SO₃⁻, R"HPO₃⁻, R"'PO₃²⁻, R""CO₂⁻ bedeutet, oder Mischungen davon, eingesetzt werden. Bevorzugt sind Mineralsäuren, insbesondere HCl, gasförmig oder in einem Lösungsmittel, vorzugsweise Wasser oder Alkohol. Der im erfindungsgemäßen Verfahren verwendete Säure-Katalysator wird In dem Fachmann hierfür gebräuchlichen Mengen eingesetzt.

Basierend auf dem Standard-Verfahren von Gassman et. al. (J. Am. Chem. Soc., 1974, 96(17), 5508) sind mehrere Oxindole oder ortho-substituierte Aniline synthetisiert worden. So wurde z.B. auf dem Weg der Synthese eines 7-Fluorisatins das Zwischenprodukt 7-Fluor-3-methylthio-oxindol synthetisiert (Wierenga et al., Tetrahedron Lett. 1983, 24,2437). Ein Beispiel für ein nach dem Gassman-Verfahren unter Verwendung von Proton Sponge und Triethylamin hergestelltes 7-Fluor-3-mathylthio-4-nitro-oxindol findet sich in Tetrahedron Lett. 2005, 46, 4613.

Im Zusammenhang mit den in dieser Beschreibung verwendeten chemischen Begriffen gelten die für den Fachmann üblichen Definitionen, sofern nichts anderes spezifisch definiert ist. Die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C Atomen bzw. bei ungesättigten Gruppen mit 2 bis 6 C Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl.

Cycloalkyl bedeutet ein carbocyclisches, gesättigtes Ringsystem mit vorzugsweise 3 bis 8 C Atomen, z.B. Cyclopropyl, Cyclobutyl, Cyclopentyl oder Cyclohexyl.

Halogen bedeutet beispielsweise Fluor, Chlor, Brom oder Iod. Haloalkyl bedeutet durch Halogen, vorzugsweise durch Fluor, Chlor und/oder Brom, insbesondere durch Fluor und/oder Chlor, teilweise oder vollständig substituiertes Alkyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; er enthält vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono , bi oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Oxazolinyl, Isoxazolinyl, Oxazolidinyl, Isoxazolidinyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie ein substituierter Alkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Sulfamoyl, Mono und Dialkylaminosulfonyl, substituiertes Amino, wie Acylamino, Mono und Dialkylamino, und Alkylsulfonyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Phenyl, Phenoxy etc. eingeschlossen. Bei Resten mit C Atomen sind solche mit 1 bis 4 C Atomen, insbesondere 1 oder 2 C Atomen, bevorzugt. Bevorzugt sind in der Regel Substituenten aus der Gruppe Halogen, z.B. Fluor und Chlor, (C1-C4)Alkyl, vorzugsweise Methyl oder Ethyl, (C1-C4)Haloalkyl, vorzugsweise Trifluormethyl, (C1-C4)Alkoxy, vorzugsweise Methoxy oder Ethoxy, (C1-C4)Haloalkoxy, Nitro und Cyano. Besonders bevorzugt sind dabei die Substituenten Methyl und Fluor.

Von den Formeln (4'), (7') und (8') sind, soweit zutreffend, auch alle Stereoisomeren umfasst. Solche Verbindungen enthalten ein oder mehrere asymmetrische C-Atome, die in den allgemeinen Formeln nicht gesondert angegeben sind. Die durch ihre spezifische Raumform definierten möglichen Stereoisomeren, wie Enantiomere, Diastereomere, können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die erfindungsgemäß einzusetzenden Verbindungen der Formeln Q und W sind bekannt oder können analog zu allgemein bekannten Verfahren hergestellt werden.

Das erfindungsgemäße Verfahren zum Erhalt von Verbindungen der Formel (4) wird beispielsweise so durchgeführt, dass man Anilin (Verbindung der Formel Q) und Thioether (Verbindung der Formel W) im Lösungsmittel vorlegt und, vorzugsweise unter Schutzgas, herunterkühlt. Zu dieser gerührten Lösung wird eine Mischung aus Chlorierungsmittel und Lösungsmittel zugetropft. Die Reaktion wird anschließend erwärmt und mit einer wässrigen Säure verdünnt. Die Phasen werden getrennt und die organische Phase mit einer wässrigen Säure gewaschen. Die organische Phase wird anschließend getrocknet und im Falle, dass die Verbindung der Formel (4) ein Feststoff ist, *in vacuo* aufkonzentriert und möglicherweise ein Antilösungsmittel zugegeben und mehrere Stunden gerührt. Der Feststoff (Verbindung der Formel (4)) wird abfiltriert und mit dem Antilösungsmittel oder Lösungsmittelgemisch gewaschen. Im Falle, dass die Verbindung der Formel (4) ein Öl ist, wird die organische Phase *in vacuo* aufkonzentriert.

Das erfindungsgemäße Verfahren zum Erhalt von Verbindungen der Formeln (7) und (8) wird beispielsweise so durchgeführt, dass man Anilin (Verbindung der Formel Q) und Thioether (Verbindung der Formel W) im Lösungsmittel vorlegt und, vorzugsweise unter Schutzgas, herunterkühlt. Zu dieser gerührten Lösung wird eine Mischung aus Chlorierungsmittel und Lösungsmittel zugetropft. Die Reaktion wird anschließend erwärmt und mit einer wässrigen Säure verdünnt. Die Phasen werden getrennt und die organische Phase mit einer wässrigen Säure gewaschen. Die organische Phase wird anschließend, möglicherweise für Verbindungen der Formel (7) und vorzugsweise für Verbindungen der Formel (8), mit einem Säurekatalysator versetzt und mehrere Stunden gerührt. 90% des Lösungsmittels wird anschließend *in vacuo* entfernt und ein Antilösungsmittel zugegeben und mehrere Stunden gerührt. Der Feststoff (Verbindung der Formel (7) oder (8)) wird abfiltriert und mit dem Antilösungsmittel oder Lösungsmittelgemisch gewaschen.

Die folgenden Beispiele erläutern das erfindungsgemäße Verfahren näher, ohne das erfindungsgemäße Verfahren darauf zu beschränken. In den folgenden Beispielen beziehen sich Mengenangaben auf das Gewicht, sofern nichts anderes speziell definiert ist (in der Beschreibung wurde hierfür analog Gew.% = Gewichtsprozent verwendet). Für Maßeinheiten, physikalische Größen und ähnliches werden übliche Abkürzungen verwendet, beispielsweise h = Stunde(n), mpt. = Schmelzpunkt (Smp.), I = Liter, ml = Milliliter, g = Gramm, min = Minute(n), *in vacuo* = "im Vakuum" = unter reduziertem Druck, d. Th. = Prozent Ausbeute nach der Theorie.

### Beispiele

### Synthesebeispiel 1:

2-Chloranilin (20,4g) und Methylmercaptoessigsäure-methylester (8,36g) wurden in Chlorbenzol (77ml) vorgelegt und unter Schutzgas auf -30°C gekühlt. Zu dieser gerührten Lösung wurde eine Lösung von Sulfurylchlorid (8,45g) in Chlorbenzol (67ml) in 30 Minuten zugetropft. Die Reaktionsmischung wurde auf -5°C erwärmt und mit 0,4N Salzsäure (110ml) verdünnt. Die Phasen wurden getrennt und die organische Phase mit 0,4N Salzsäure gewaschen (4 x 110ml). Die organische Phase wurde dann mit 10%iger HCl in MeOH (2ml) versetzt und 16 Stunden gerührt. 90% des Lösungsmittels wurden *in vacuo* entfernt, Heptan (120ml) zugegeben und 4 Stunden gerührt. Der Feststoff wurde abfiltriert und mit Heptan (2 x 50ml) gewaschen. Man erhält 7-Chlor-3-methylthio-oxindol (9,36g 70% d. Th.). mpt.: 167-170°C.

### Synthesebeispiel 2:

2-Chloranilin (20,4g) und Methylmercaptoessigsäure-methylester (8,36g) wurden in Chlorbenzol (77ml) vorgelegt und unter Schutzgas auf -30°C gekühlt. Zu dieser gerührten Lösung wurde eine Lösung von Sulfurylchlorid (8,45g) in Chlorbenzol (67ml) in 30 Minuten zugetropft. Die Reaktionsmischung wurde auf -5°C erwärmt und mit 0,4N Salzsäure (110ml) verdünnt. Die Phasen wurden getrennt, die organische Phase mit 0,4N Salzsäure (4 x 110ml) und Wasser (50ml) gewaschen und über Natriumsulfat getrocknet. Die organische Phase wurde filtriert und *in vacuo* abdestilliert. Man erhält (2-Amino-3-chlophenyl)-(methylthio)essigsäure-methylester als braun-rotes Öl (10,9 g 71% d.Th.).

### Synthesebeispiel 3:

4-Chloranilin (20,4g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) in 30 Minuten zugetropft. Die Reaktion wurde auf 10°C erwärmt und mit 0,4N Salzsäure (110ml) verdünnt. Die Phasen wurden getrennt und die organische Phase mit 0,4N Salzsäure gewaschen (2 x 110ml). Die organische Phase wurde dann mit 10%iger HCl in MeOH (2ml) versetzt und 16 Stunden gerührt. 90% des Lösungsmittels wurden *in vacuo* entfernt, Heptan (120ml) zugegeben und 4 Stunden gerührt. Der Feststoff wurde abfiltriert und mit Heptan (2 x 50ml) gewaschen. Man erhält 5-Chlor-3-methylthio-oxindol (7,89g 59% d. Th.). mpt.: 154-157°C.

### Synthesebeispiel 4:

2-Fluoranilin (17,8g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) in 30 Minuten zugetropft. Die Reaktion wurde auf 10°C erwärmt und mit 0,4N Salzsäure (110ml) verdünnt. Die Phasen wurden getrennt und die organische Phase mit 0,4N Salzsäure gewaschen (1 x 110ml). Die organische Phase wurde dann mit 10%iger HCl in MeOH (2ml) versetzt und 16 Stunden gerührt 90% des Lösungsmittels wurden *in vacuo* entfernt und Heptan (120ml) zugegeben und 4 Stunden gerührt. Der Feststoff wurde abfiltriert und mit Heptan (2 x 50ml) gewaschen. Man erhält 7-Fluor-3-methylthio-oxindol (9,17g 72% d. Th.). mpt.: 156-159°C.

### Synthesebeispiel 5:

2-Fluoranilin (17,8g) und Methylmercaptoessigsäure-methylester (8,36g) wurden in Essigsäure-n-butylester (77ml) vorgelegt und unter Schutzgas auf -30°C gekühlt. Zu dieser gerührten Lösung wurde eine Lösung von Sulfurylchlorid (8,45g) in Essigsäure-n-butylester (67ml) In 30 Minuten zugetropft. Die Reaktion wurde auf 10°C erwärmt und mit 0,4N Salzsäure (110ml) verdünnt. Die Phasen wurden getrennt, die organische Phase mit 0,4N Salzsäure (1 x 110ml) und Wasser (50ml) gewaschen und über Natriumsulfat getrocknet. Die organische Phase wurde filtriert und *in vacuo* abdestilliert. Man erhält (2-Amino-3-fluorphenyl)-(methylthio)essigsäure-methylester als braunes Öl (12,5 g 73% d.Th.).

### Synthesebeispiel 6:

2-Fluoranilin (17,8g) und Methylmercaptoessigsäure-methylester (8,36g) wurden in Essigsäure-n-butylester (77 ml) vorgelegt und unter Schutzgas auf -50°C gekühlt. Zu dieser gerührten Lösung wurde eine Lösung von Sulfurylchlorid (8,45g) in Essigsäure-n-butylester (67 ml) in 30 Minuten bei einer Innentemperatur von -53 bis -48°C zugetropft. Die Reaktion wurde auf 10°C erwärmt, mit 0,4N Salzsäure (110ml) verdünnt und 5 min nachgerührt. Die Phasen wurden getrennt und die organische Phase mit 0,4N Salzsäure gewaschen (1 x 110ml). Die organische Phase wurde mit konz. HCl (0.2 ml) versetzt. Die Reaktionsmischung wurde 24 Stunden gerührt, noch einmal mit konz. HCl (0.05 ml) versetzt und 16 Stunden bei 4°C stehen gelassen. Ungefähr 90% des Lösungsmittels wurden *in vacuo* entfernt, Heptan (120ml) zugegeben und 5 Stunden gerührt. Der Feststoff wurde abfiltriert, mit Heptan (2 x 50ml) gewaschen und getrocknet Man erhält 7-Fluor-3-methylthio-oxindol (8.71g 63% d. Th.). LCMS: M+H = 198 (100%). Das 1H-NMR stimmt mit dem in Synthesebeispiel 7 beschriebenen überein.

### Synthesebeispiel 7:

2-Fluoranilin (18,1g) und Methylmercaptoessigsäure-methylester (8,58g) wurden in Essigsäure-n-butylester (80 ml) vorgelegt und unter Schutzgas auf -20°C gekühlt. Zu dieser gerührten Lösung wurde eine Lösung von Sulfurylchlorid (8,77g) in Essigsäure-n-butylester (70 ml) in 30 Minuten bei einer Innentemperatur von -25 bis -18°C zugetropft. Die Reaktion wurde innerhalb 60 min auf 10°C erwärmt und mit 0,4N Salzsäure (110ml) verdünnt Die Phasen wurden getrennt und die organische Phase mit 0,4N Salzsäure gewaschen (1 x 110ml). Die organische Phase wurde dann mit konz. Salzsäure (0.5 ml) und MeOH (2ml) versetzt und 16 Stunden gerührt. Ungefähr 90% des Lösungsmittels wurden *in vacuo* entfernt, n-Heptan (120 ml) zugegeben und 3 Stunden gerührt. Der Feststoff wurde abfiltriert und mit Heptan gewaschen. Man erhält 7-Fluor-3-methylthio-oxindol (7.85g, 55% d. Th.). 1 H-NMR (CDCl₃): d = 2.06 (s, 3H), 4.32 (s, 1H), 7.02-7.05 (m, 2H), 7.17-7.19 (m, 1H), 8.3 (s, breit, 1 H).

### Synthesebeispiel 8:

2-Fluoranilin (17,8g) und Methylmercaptoessigsäure-methylester (8,36g) wurden in Dichlormethan (77 ml) vorgelegt und unter Schutzgas auf -30°C gekühlt. Zu dieser gerührten Lösung wurde eine Lösung von Sulfurylchlorid (8,45g) in Essigsäure-n-butylester (67 ml) in 30 Minuten bei einer Innentemperatur von -33 bis -28°C zugetropft. Die Reaktion wurde auf 10°C erwärmt, mit 0,4N Salzsäure (110ml) verdünnt und 5 min nachgerührt. Die Phasen wurden getrennt und die organische Phase mit 0,4N Salzsäure gewaschen (1 x 110ml). Die organische Phase wurde dann mit konz. HCl (0.2 ml) versetzt. Die Reaktionsmischung wurde 16 Stunden gerührt, mit konz. HCl (0.05 ml) versetzt, 7 Stunden gerührt, noch einmal mit konz. HCl (0.05 ml) versetzt und 16 Stunden nachgerührt. Ungefähr 90% des Lösungsmittels wurden *in vacuo* entfernt, Heptan (110 ml) zugegeben und 5 Stunden gerührt. Der Feststoff wurde abfiltriert, mit Heptan (2 x 50ml) gewaschen und getrocknet Man erhält 7-Fluor-3-methylthio-oxindol (5.84g 42% d. Th.). LCMS und 1 H-NMR stimmen mit den in Synthesebeispielen 6 und 7 beschriebenen überein.

### Synthesebeispiel 9:

2-Fluoranilin (7,73g) und Methylmercaptoessigsäuremethylester (8,36g) wurden in Essigsäure-n-butylester (67ml) vorgelegt und unter Schutzgas auf -35°C gekühlt. Zu dieser gerührten Lösung wurde eine Lösung von Sulfurylchlorid (8,45g) in Essigsäure-n-butylester (67ml) und parallel dazu 2-Fluoranilin (10,04g) in Essigsäure-n-butylester (10ml) in 25 Minuten direkt In die Reaktionslösung zudosiert, wobei die Temperatur auf -29°C anstieg. Nach 15 Minuten bei -30°C wurde die Reaktion auf 10°C erwärmt und 0,4N Salzsäure (110ml) zugetropft. Die Phasen wurden getrennt und die organische Phase mit 0,4N Salzsäure gewaschen (1 x 110ml). Die organische Phase wurde mit 10%iger HCl in MeOH (1,9ml) versetzt und 16 Stunden gerührt. 95% des Lösungsmittels wurden im Vakuum entfernt, Heptan (120ml) zugegeben und die Reaktionsmischung 3 Stunden gerührt. Der Feststoff wurde abfiltriert und mit Heptan (2 x 50ml) gewaschen. Man erhält 7-Fluor-3-methylthio-oxindol (8,87g 70,0% d. Th.).

### Synthese beispiel 10:

2-Fluoranilin (18.1g) und 1-(Methylsulfanyl)aceton (7.44g) wurden in Essigsäure-n-butylester (80 ml) vorgelegt und unter Schutzgas auf -30°C gekühlt. Unter Rühren wurde eine Lösung von Sulfurylchlorid (8,77g) in Essigsäure-n-butylester (70 ml) innerhalb 30 Minuten zugetropft und 1.5 Stunden bei einer Innentemperatur von -35 bis -28°C nachgerührt. Die Reaktionsmischung wurde auf 0°C erwärmt und die Phasen getrennt. Die organische Phase wurde mit 0.5N Salzsäure (2 mal 80 ml) und Wasser (40 ml) extrahiert, mit etwas Natriumsulfat getrocknet und im Vakuum eingeengt. Der Rückstand wird dreimal mit Ethylacetat versetzt und eingeengt. Man erhält 7-Fluor-2-methyl-3-(methylsulfanyl)-1H-indol als braunes Öl (9.67g, 59% d. Th.). 1H-NMR (CDCl₃): d = 2.26 (s, 3H), 2.55 (s, 3H), 6.87 (dd, 1 H), 7.03-7.08 (m, 1 H), 7.43 (d, 1 H), 8.24 (s, breit, 1 H). LCMS: M+H = 196.

## Patentansprüche

1. Verfahren zur Herstellung von Verbindungen der Formel (4): 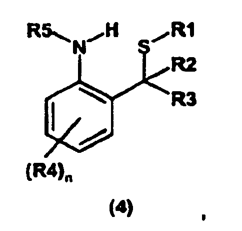 worin
R1 = C1-C6 Alkyl, substituiertes Alkyl, Aryl oder substituiertes Aryl,
vorzugsweise C1-C4 Alkyl;
R2 = H, C1-C6 Alkyl oder substituiertes Alkyl;
R3 = elektronenziehende oder aktivierende Gruppe, ausgewählt aus -CO-R1; -CO-X,
wobei X = OR1, SR1, NR2R2', wobei R2' wie R2 definiert ist und gleich oder anders als R2 sein kann; R2 und R2' können einen Ring bilden; SO(n')-R1, wobei n' = 0, 1, oder 2 sein kann; -CN; -NO₂, Aryl or Heteroaryl;
R4 = F, Cl, Br, I, CF₃, CN, NO₂, COX;
wobei X = OR1, SR1, NR2R2', wobei R2' wie R2 oben definiert ist und gleich oder anders als R2 sein kann, vorzugsweise F oder Cl, insbesondere 2-F;
n = 1 - 4, vorzugsweise 1 - 2, insbesondere 1;
R5 = H, C1-C6 Alkyl oder substituiertes Alkyl,
bedeutet,
**dadurch gekennzeichnet, dass** man eine Mischung
eines Anilins (Verbindung der Formel Q): 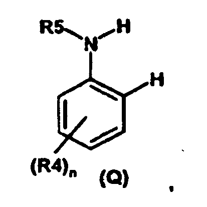 worin die Reste R4, n und R5 wie in Formel (4) definiert sind,
mit einem Thioether (Verbindung der Formel W): 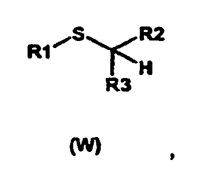 worin die Reste R1, R2 und R3 wie in Formel (4) definiert sind,
in Gegenwart von Sulfurylchlorid (SO₂Cl₂) als Chlorierungsmittel und eines organischen Lösungsmittels bei einer Reaktionstemperatur im Bereich zwischen -60 und -10°C, insbesondere zwischen -50 und -20°C, zur Verbindung der Formel (4) umsetzt.

2. Verfahren nach Anspruch 1, **dadurch gekennzeichnet, dass** die Reaktion in Ester-Lösungsmitteln ausgewählt aus der Gruppe bestehend aus C1-C6 Alkylacetate und Cyclalkylacetate, C1-C6 Alkyl und Cycloalkylpropionate, C1-C6 Alkyl und Cycloalkyl -n-butyrate, -iso-butyrate, -pentanoate und - hexanoate und -cyclopentanoate und -cyclohexanoate oder in Mischungen davon oder in Mischung mit anderen Lösungsmitteln, durchgeführt wird.

3. Verfahren nach Anspruch 1 oder 2, **dadurch gekennzeichnet, dass** für gute Produktausbeuten bis zu 1 Äquivalent des Chlorierungsmittels zu einer Mischung eines Äquivalents des Thioethers (W) und 2,0 bis 5,0 Äquivalente, des Anilins (Q) zugegeben wird.

4. Verfahren nach einem der Ansprüche 1 bis 3, **dadurch gekennzeichnet, dass** eine Teilmenge des Anilins zwischen 1 und 99 Gew.% der Gesamtmenge an Anilin getrennt vom, aber simultan oder teilweise simultan mit dem Chlorierungsmittel zugegeben wird.

5. Verfahren nach einem der Ansprüche 1 bis 4, **dadurch gekennzeichnet, dass** auf die Zugabe eines zusätzlichen tertiären Amins verzichtet wird.

6. Verfahren zur Herstellung von Verbindungen der Formeln (7) und (8): 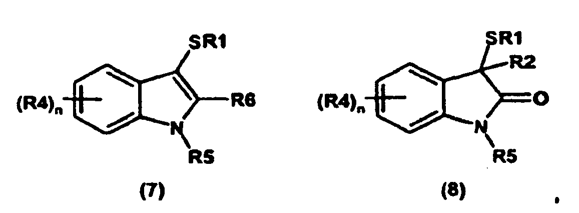 worin
R1 = C1-C6 Alkyl, substituiertes Alkyl, Aryl oder substituiertes Aryl,
vorzugsweise C1-C4 Alkyl;
R2 = H, C1-C6 Alkyl oder substituiertes Alkyl;
R4 = F, Cl, Br, I, CF₃, CN, NO₂, COX;
wobei X = OR1, SR1, NR2R2', wobei R2' wie R2 oben definiert ist und gleich oder anders als R2 sein kann, vorzugsweise F oder Cl, insbesondere 2-F;
n = 1 - 4, vorzugsweise 1 - 2 , insbesondere 1;
R5 = H, C1-C6 Alkyl oder substituiertes Alkyl;
R6 = C1-C6 Alkyl oder substituiertes Alkylvorzugsweise C1-C4 Alkyl oder OH;
bedeutet,
wobei Verbindungen der Formel (4): 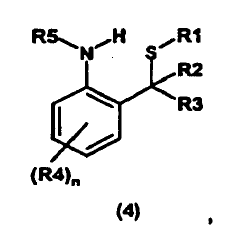 mit den oben genannten Definitionen der Reste R1, R2, R4, n und R5 un wobei R3 eine elektronenziehende oder aktivierende Gruppe, ausgewählt aus -CO-R1; - CO-X, wobei X = OR1, SR1, NR2R2', wobei R2' wie R2 definiert ist und gleich oder anders als R2 sein kann; R2 und R2' können einen Ring bilden; SO(n')-R1, wobei n' = 0, 1, oder 2 sein kann; -CN; -NO₂, Aryl or Heteroaryl; bedeutet,
hergestellt nach einem Verfahren gemäß einem der Ansprüche 1 bis 5;
ohne Isolierung der Verbindungen der Formel (4), gegebenenfalls in Gegenwart eines Säure-Katalysators, zu dem Indol der Formel (7) oder dem Oxindol der Formel (8) umgesetzt werden,
wobei die Verbindung (4) dadurch hergestellt wird, dass man eine Mischung eines Anilins der Formel Q 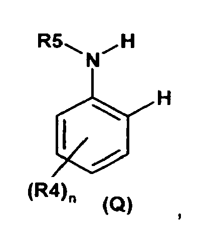 worin die Reste R4, n und R5 wie in Formel (4) definiert sind,
mit einem Thioether (Verbindung der Formel W): 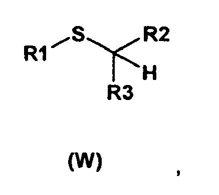 worin die Reste R1, R2 und R3 wie in Formel (4) definiert sind,
in Gegenwart von Sulfurylchlorid und eines organischen Lösungsmittels bei einer Reaktionstemperatur im Bereich zwischen -60 und -10°C, insbesondere zwischen -50 und -20°C, zur Verbindung der Formel (4) umsetzt.

## Claims

1. A process for the preparation of compounds of formula (4), 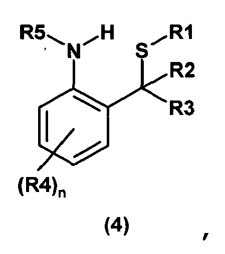 in which
R1 = C1-C6 alkyl, substituted alkyl, aryl or substituted aryl, preferably C1-C4 alkyl;
R2 = H, C1-C6 alkyl or substituted alkyl;
R3 = electron-withdrawing or activating group, selected from -CO-R1; -CO-X, where X = OR1, SR1, NR2R2', where R2' is defined like R2 and may be identical to or different from R2; R2 and R2' can form a ring; SO(n')-R1, where n' may be 0, 1 or 2; -CN; -NO₂, aryl or heteroaryl;
R4 = F, Cl, Br, I, CF₃, CN, NO₂, COX; where X = OR1, SR1, NR2R2', where R2' is defined like R2 above and may be identical to or different from R2, preferably F or Cl, in particular 2-F;
n = 1 - 4, preferably 1 - 2, in particular 1;
R5 = H, C1-C6 alkyl or substituted alkyl,
which comprises reacting a mixture
of an aniline (compound of formula Q): 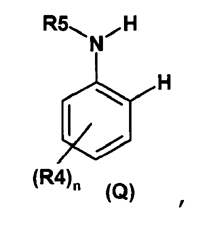 in which the radicals R4, n and R5 are as defined in formula (4),
with a thioether (compound of formula W): 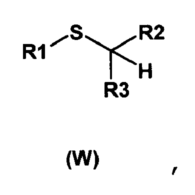 in which the radicals R1, R2 and R3 are as defined in formula (4),
in the presence of sulfuryl chloride (SO₂Cl₂) as chlorinating agent and an organic solvent at a reaction temperature in the range between -60 and -10°C, in particular between -50 and -20°C, to give the compound of formula (4).

2. The process as claimed in claim 1, wherein the reaction is carried out in ester solvents selected from the group consisting of C1-C6 alkyl acetates and cycloalkyl acetates, C1-C6 alkyl and cycloalkyl propionates, C1-C6 alkyl and cycloalkyl n-butyrates, isobutyrates, pentanoates and hexanoates and cyclopentanoates and cyclohexanoates or in mixtures thereof or in a mixture with other solvents.

3. The process as claimed in claim 1 or 2, wherein, for good product yields, up to 1 equivalent of the chlorinating agent is added to a mixture of one equivalent of the thioether (W) and 2.0 to 5.0 equivalents of the aniline (Q).

4. The process as claimed in any of claims 1 to 3, wherein a part amount of the aniline between 1 and 99% by weight of the total amount of aniline is added separately from, but simultaneously or partially simultaneously with the chlorinating agent.

5. The process as claimed in any of claims 1 to 4, wherein the addition of an additional tertiary amine is dispensed with.

6. A process for the preparation of compounds of formulae (7) and (8): 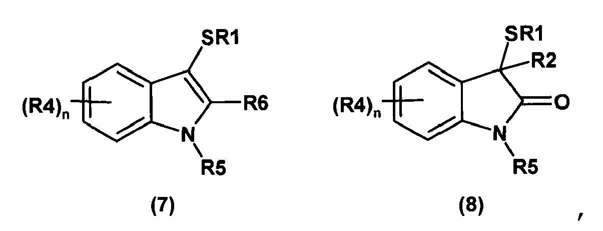 in which
R1 = C1-C6 alkyl, substituted alkyl, aryl or substituted aryl, preferably C1-C4 alkyl;
R2 = H, C1-C6 alkyl or substituted alkyl;
R4 = F, Cl, Br, I, CF₃, CN, NO₂, COX; where X = OR1, SR1, NR2R2', where R2' is defined like R2 above and may be identical to or different from R2, preferably F or Cl, in particular 2-F;
n = 1 - 4, preferably 1 - 2, in particular 1;
R5 = H, C1-C6 alkyl or substituted alkyl;
R6 = C1-C6 alkyl or substituted alkyl, preferably C1-C4 alkyl or OH;
where compounds of formula (4): 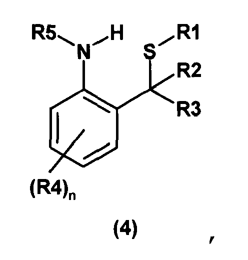 with the aforementioned definitions of the radicals R1, R2, R4, n and R5 and where R3 is an electron-withdrawing or activating group, selected from -CO-R1; -CO-X, where X = OR1, SR1, NR2R2', where R2' is defined like R2 and may be identical to or different from R2; R2 and R2' can form a ring; SO(n')-R1, where n' may be 0, 1 or 2; -CN; -NO₂, aryl or heteroaryl;
prepared by a process as claimed in one of claims 1 to 5;
are reacted without isolation of the compounds of formula (4), optionally in the presence of an acid catalyst, to give the indole of formula (7) or the oxindole of formula (8),
where the compound (4) is prepared by reacting a mixture of an aniline of formula Q 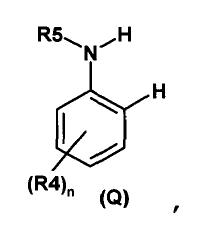 in which the radicals R4, n and R5 are as defined in formula (4),
with a thioether (compound of formula W): 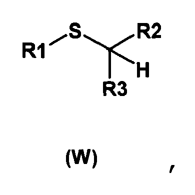 in which the radicals R1, R2 and R3 are as defined in formula (4),
in the presence of sulfuryl chloride and an organic solvent at a reaction temperature in the range between -60 and -10°C, in particular between -50 and -20°C, to give the compound of formula (4).

## Revendications

1. Procédé pour la préparation de composés de formule (4) : 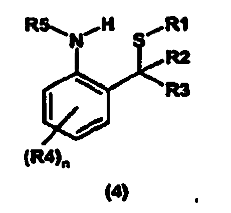 dans laquelle
R1 = C1-C6-alkyle, alkyle substitué, aryle ou aryle substitué, de préférence C1-C4-alkyle ;
R2 = H, C1-C6-alkyle ou alkyle substitué ;
R3 = un groupe attracteur d'électrons ou d'activation, choisi parmi -CO-R1 ; -CO-X, X = OR1, SR1, NR2R2', R2' étant défini comme R2 et pouvant être identique ou différent de R2 ; R2 et R2' peuvent former un cycle ; SO(n')-R1, n' pouvant être 0, 1 ou 2 ; -CN ; -NO₂, aryle ou hétéroaryle ;
R4 = F, Cl, Br, I, CF₃, CN, NO₂, COX ;
X = OR1, SR1, NR2R2', R2' étant défini comme R2 ci-dessus et pouvant être identique ou différent de R2, de préférence F ou Cl, en particulier 2-F ;
n = 1-4, de préférence 1-2, en particulier 1 ;
R5 = H, C1-C6-alkyle ou alkyle substitué
**caractérisé en ce qu'**on transforme un mélange d'une aniline (composé de formule Q) 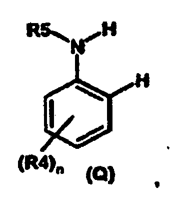 les radicaux R4, n et R5 étant définis comme dans la formule (4), avec un thioéther (composé de formule W) : 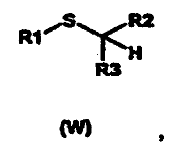 les radicaux R1, R2 et R3 étant définis comme dans la formule (4),
en présence de chlorure de sulfuryle (SO₂Cl₂) comme agent de chloration et d'un solvant organique à une température de réaction dans la plage entre -60 et -10°C, en particulier entre -50 et -20°C, en composé de formule (4).

2. Procédé selon la revendication 1, **caractérisé en ce que** la réaction est réalisée dans un solvant de type ester, choisi dans le groupe constitué par les acétates de C1-C6-alkyle et les acétates de cycloalkyle, les propionates de C1-C6-alkyle et de cycloalkyle, les n-butyrates, les isobutyrates, les pentanoates et les hexanoates et les cyclopentanoates et les cyclohexanoates de C1-C6-alkyle et de cycloalkyle ou dans des mélanges de ceux-ci ou en mélange avec d'autres solvants.

3. Procédé selon la revendication 1 ou 2, **caractérisé en ce qu'**on ajoute, pour de bons rendements de produit, jusqu'à 1 équivalent de l'agent de chloration à un mélange d'un équivalent du thioéther (W) et de 2,0 à 5,0 équivalents de l'aniline (Q).

4. Procédé selon l'une quelconque des revendications 1 à 3, **caractérisé en ce qu'**une quantité partielle de l'aniline entre 1 et 99% en poids de la quantité totale d'aniline est ajoutée séparément de, mais simultanément ou de manière partiellement simultanée avec, l'agent de chloration.

5. Procédé selon l'une quelconque des revendications 1 à 4, **caractérisé en ce qu'**on renonce à l'addition d'une amine tertiaire supplémentaire.

6. Procédé pour la préparation de composés des formules (7) et (8) : 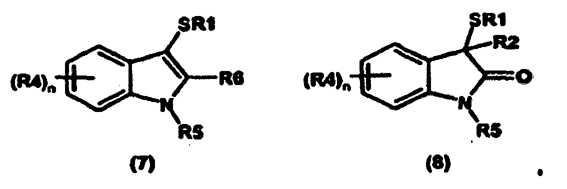 dans lesquelles
R1 = C1-C6-alkyle, alkyle substitué, aryle ou aryle substitué, de préférence C1-C4-alkyle ;
R2 = H, C1-C6-alkyle ou alkyle substitué ;
R4 = F, Cl, Br, I, CF₃, CN, NO₂, COX ;
X = OR1, SR1, NR2R2', R2' étant défini comme R2 ci-dessus et pouvant être identique ou différent de R2, de préférence F ou Cl, en particulier 2-F ;
n = 1-4, de préférence 1-2, en particulier 1 ;
R5 = H, C1-C6-alkyle ou alkyle substitué ;
R6 = C1-C6-alkyle ou alkyle substitué, de préférence C1-C4-alkyle ou OH ;
des composés de formule (4) : 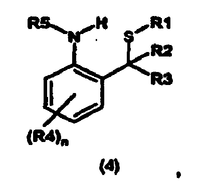 présentant les définitions susmentionnées des radicaux R1, R2, R4, n et R5, et R3 signifiant un groupe attracteur d'électrons ou d'activation, choisi parmi -CO-RL ; -CO-X, X = OR1, SR1, NR2R2', R2' étant défini comme R2 et pouvant être identique ou différent de R2 ;
R2 et R2' peuvent former un cycle ; SO(n')-R1, n' pouvant être - 0, 1 ou 2 ; -CN ; -NO₂, aryle ou hétéroaryle ;
préparés selon un procédé selon l'une quelconque des revendications 1 à 5 ;
étant transformés sans isolement des composés de formule (4), le cas échéant en présence d'un catalyseur acide, en indole de formule (7) ou en oxindole de formule (8),
le composé (4) étant préparé en ce qu'on transforme une aniline de formule Q 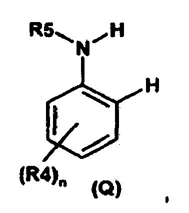 les radicaux R4, n et R5 étant définis comme dans la formule (4),
avec un thioéther (composé de formule W) : 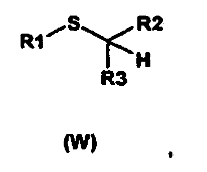 les radicaux R1, R2 et R3 étant définis comme dans la formule (4),
en présence de chlorure de sulfuryle et d'un solvant organique à une température de réaction dans la plage entre -60 et -10°C, en particulier entre -50 et -20°C, en composé de formule (4).
